# FASCICULE DE BREVET EUROPEEN

(11) **EP 0 818 437 B1**
(45) Date de publication et mention de la délivrance du brevet: **15.03.2000**
(21) Numéro de dépôt: 97401497.9
(22) Date de dépôt: 26.06.1997
(51) Int. Cl.: C07C 219/08, C07C 213/08

(54) **Procédé de fabrication de solutions aqueuses de sels d'ammonium quaternaire insaturés**
Verfahren zur Herstellung von wässrigen Lösungen von ungesättigten quaternären Ammoniumsalzen
Process for the preparation of aqueous solutions of insaturated quaternary ammonium salts

(30) Priorité: 08.07.1996 FR 9608477
(43) Date de publication de la demande: 14.01.1998
(73) Titulaire: ELF ATOCHEM S.A., 92800 Puteaux, Hauts-de-Seine (FR)
(72) Inventeur: Riondel, M. Alain, 57600 Forbach (FR)
(74) Mandataire: Rieux, Michel

(56) Documents cités:
- EP-A- 0 329 512
- FR-A- 2 706 453
- PATENT ABSTRACTS OF JAPAN vol. 006, no. 202 (C-129), 13 Octobre 1982 & JP 57 109747 A (SANYO KASEI KOGYO KK), 8 Juillet 1982
- PATENT ABSTRACTS OF JAPAN vol. 018, no. 097 (C-1167), 17 Février 1994 & JP 05 295011 A (KURARAY CO LTD), 9 Novembre 1993
- PATENT ABSTRACTS OF JAPAN vol. 006, no. 202 (C-129), 13 Octobre 1982 & JP 57 109749 A (SANYO KASEI KOGYO KK), 8 Juillet 1982
- DATABASE WPI Section Ch, Week 7643 Derwent Publications Ltd., London, GB; Class A14, AN 76-80482X XP002026796 & JP 51 103 185 A (MITSUBISHI CHEM IND KK) , 12 Septembre 1976
- PATENT ABSTRACTS OF JAPAN vol. 007, no. 118 (C-167), 21 Mai 1983 & JP 58 037058 A (SUMITOMO KAGAKU KOGYO KK), 4 Mars 1983

## Description

La présente invention porte sur la fabrication d'une solution aqueuse du sel d'ammonium quaternaire insaturé, répondant à la formule (I) suivante : 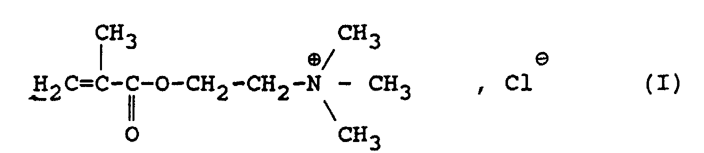 par réaction, en présence d'eau, du méthacrylate de N,N-diméthylaminoéthyle avec CH₃-Cl comme agent quaternisant.

Les solutions aqueuses de ce sel (I) sont utilisées pour préparer des polymères destinés à servir de floculants dans le traitement des eaux.

A l'heure actuelle, on est contraint de conduire le procédé ci-dessus à la pression atmosphérique, car la synthèse sous pression conduit à un produit instable thermiquement. Cependant, le procédé actuel sous pression atmosphérique présente le désavantage d'utiliser un excès de CH₃-Cl plus important - dont la conséquence directe est un rejet gazeux de CH₃-Cl plus important - que si la synthèse était effectuée sous pression.

Par ailleurs, un procédé de préparation du sel (I) sous pression est connu par la demande de brevet japonais J 5 76-103 185. Conformément à ce procédé connu, une solution aqueuse à 50-80% (pp) d'un (méth)acrylate de formule : 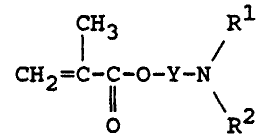 dans laquelle :
- Y représente un groupe alkylène inférieur ou hydroxyalkylène inférieur ; et
- R¹ et R² représentent chacun indépendamment un groupe alkyle inférieur
est amenée en contact avec un halogénure d'alkyle ou d'alkaryle pour quaterniser le méthacrylate ci-dessus. Le seul exemple illustrant cette quaternisation est effectué avec addition de chlorure de méthyle sous une pression maximale de 4,5 bars, à une température de 25-30°C. En dehors de l'inconvénient indiqué ci-dessus concernant le rejet du chlorure de méthyle en excès, ce procédé conduit à une hydrolyse importante du composé de départ, par exemple du méthacrylate de N,N-diméthylaminoéthyle en acide méthacrylique, cette réaction parasite étant favorisée par le mode d'introduction du méthacrylate de N,N-diméthylaminoéthyle et de l'eau qui sont ajoutés déjà mélangés et en début de réaction. Cette présence d'acide méthacrylique dans la solution aqueuse finale obtenue dégrade la qualité analytique de celle-ci, et, peut, dans certaines applications, avoir une influence néfaste sur sa valeur d'usage en polymérisation.

La présente invention a pour but de remédier aux inconvénients précités.

De façon surprenante, la Société déposante a maintenant découvert que le procédé sous pression pouvait conduire à un produit thermiquement stable, à la condition qu'il soit effectué en présence d'un composé particulier.

La présente invention a donc d'abord pour objet un procédé de fabrication d'une solution aqueuse du sel d'ammonium quaternaire insaturé de la formule (I) telle que définie ci-dessus, par réaction, en présence d'eau, du méthacrylate de N,N-diméthylaminoéthyle avec CH₃-Cl comme agent quaternisant, ledit procédé étant conduit sous pression, caractérisé par le fait qu'il est conduit en présence de sel pentasodique de l'acide diéthylène triamine pentaacétique.

On introduit le sel pentasodique de l'acide diéthylène triamine pentaacétique généralement à raison de 1 à 100 ppm, de préférence, de 5 à 30 ppm par rapport à la solution aqueuse de sel (I).

Ledit sel pentasodique est préférentiellement introduit au début de la réaction.

D'une manière générale, ledit sel pentasodique est ajouté sous la forme d'une solution aqueuse, car il est principalement disponible sous cette forme. Ainsi, ledit sel pentasodique, commercialisé sous la dénomination VERSENEX 80, se présente sous la forme d'une solution aqueuse à 40% en poids environ.

La réaction selon l'invention est généralement conduite sous une pression de 2 à 9 bars, de préférence de 3 à 6 bars, et à une température de 35 à 65°C, en particulier de 40 à 55°C.

La réaction selon l'invention est par ailleurs conduite avec un rapport molaire de CH₃-Cl au méthacrylate de N,N-diméthylaminoéthyle qui est généralement de 1 à 1,1, de préférence de 1,01 à 1,05.

En outre, au moins un stabilisant, choisi notamment parmi le 3,5-ditert.-butyl-4-hydroxytoluène, l'éther méthylique d'hydroquinone, l'hydroquinone, le catéchol, le tert.-butyl catéchol et leurs mélanges, est avantageusement associé au méthacrylate de N,N-diméthylaminoéthyle dès le début de la réaction, à raison de 50 à 2000 ppm, de préférence de 100 à 1000 ppm, par rapport à la solution aqueuse de sel (I).

Une mise en oeuvre particulièrement préférée du procédé selon l'invention est la suivante :
(a) dans un réacteur fermé, qui contient le méthacrylate de N,N-diméthylaminoéthyle et le sel pentasodique de l'acide diéthylène triamine pentaacétique et qui a été pressurisé par de l'air de 1 à 3 bars, on introduit en continu, à la température de 40 - 55°C, 5 à 15% de la quantité pondérale nécessaire à la réaction de CH₃-Cl;
(b) ensuite, on ajoute en continu, à la température de 45 - 55°C, l'eau et le reste de CH₃-Cl jusqu'à l'obtention de la concentration souhaitée en sel (I) dans l'eau, la pression en fin de réaction pouvant atteindre 6 - 9 bars ; puis
(c) on dépressurise le réacteur et, après retour à la pression atmosphérique, on élimine le CH₃-Cl résiduaire par exemple par strippage à l'air.

Les solutions sont à des concentrations notamment d'environ 50 à 85% en poids de sel quaternaire (I) dans l'eau.

Les exemples qui vont suivre, donnés à titre indicatif, permettent de mieux comprendre l'invention. Dans ces exemples, les pourcentages indiqués sont des pourcentages en poids, et les abréviations suivantes ont été utilisées :
- MADAME : méthacrylate de diméthylaminoéthyle
- MADQUAT MC 75 : solution aqueuse de chlorure de méthacryloyloxyéthyltriméthylammonium à 75%
- EMHQ : éther méthylique de l'hydroquinone
- VERSENEX 80 : sel pentasodique de l'acide diéthylène triamine penta acétique.

### EXEMPLE 1 : Préparation de MADQUAT MC 75

Dans un réacteur en verre de 1 l, spécialement conçu pour tenir la pression (pression de service de 1,2 x 10⁶ Pa (12 bars), pression d'épreuve de 6 x 10⁶ Pa (60 bars)), équipé d'un agitateur spécifique gaz-liquide, d'une spire de refroidissement, d'une sonde de température et d'un manomètre, ainsi que d'une soupape de sécurité, on charge 0,0083 g de VERSENEX 80 (solution aqueuse à 40% de matières actives), et 471 g de MADAME stabilisé à 800 ppm d'EMHQ. Le réacteur est fermé, puis pressurisé avec 1 bar d'air.

Pour conduire la réaction de quaternisation du MADAME, on utilise 159 g de CH₃-Cl et 207,5 g d'eau, les conditions opératoires étant : [CH₃Cl]/[MADAME] = 1,05, et valeur moyenne du rapport de débit d'injection H₂O/CH₃Cl (en g/h) = 0,7.

Le milieu est porté à 47°C et on commence à introduire CH₃-Cl liquide à un débit de 40 g/h. Lorsque 10% de la totalité du chlorure de méthyle ont été introduits (soit 16 g), on commence à ajouter l'eau à un débit de 28 g/h, tout en poursuivant l'introduction du restant de chlorure de méthyle.

La durée totale d'introduction du CH₃-Cl est de 4 heures, celle de l'eau, de 7 heures. En fin de réaction, lorsque l'ajout d'eau est fini, la pression atteint 6 x 10⁵ Pa (6 bars). Le réacteur est alors dépressurisé progressivement, tout en introduisant un débit d'air, de façon à avoir un rapport gaz résiduaire/gaz entrant égal à 1 (durée : 1 heure). Après retour à la pression atmosphérique, le CH₃-Cl résiduaire est ensuite éliminé à chaud par strippage à l'air (durée - 30 minutes). On obtient ainsi 818 g de MADQUAT MC 75.

Les résultats en termes de stabilité au stockage sont rassemblés dans le Tableau 1 ci-dessous. Le test de stabilité consiste à immerger dans un bain d'huile à 92°C des tubes à essai fermés et remplis à 80% avec le produit à tester. La stabilité est appréciée en mesurant la durée avant polymérisation.

### EXEMPLE 2

On a reproduit l'Exemple 1. Les résultats sont également rapportés dans le Tableau 1.

### EXEMPLE 3 (Comparatif)

On a reproduit l'Exemple 1, mais sans VERSENEX 80. Les résultats sont également rapportés dans le Tableau 1.

**Tableau 1**

| Exemple | Quantité de VERSENEX 80 par rapport au produit final (ppm) | Stabilité (heures) |
|---|---|---|
| 1 | 10 | 162 |
| 2 | 10 | 168 |
| 3 (comparatif) | sans | 2 |

## Revendications

1. Procédé de fabrication d'une solution aqueuse du sel d'ammonium quaternaire insaturé, répondant à la formule (I) suivante : 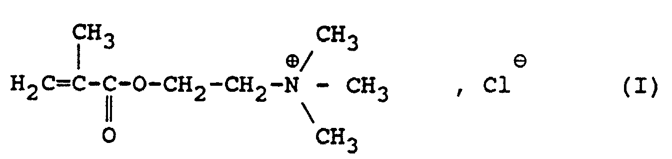 par réaction, sous pression et en présence d'eau, du méthacrylate de N,N-diméthylaminoéthyle avec CH₃-Cl comme agent quaternisant,
caractérisé par le fait qu'il est conduit en présence de sel pentasodique de l'acide diéthylène triamine pentaacétique.

2. Procédé selon la revendication 1, caractérisé par le fait qu'on introduit le sel pentasodique de l'acide diéthylène triamine pentaacétique à raison de 5 à 30 ppm par rapport à la solution aqueuse de sel (I).

3. Procédé selon l'une des revendications 1 et 2, caractérisé par le fait qu'on introduit le sel pentasodique de l'acide diéthylène triamine pentaacétique au début de la réaction.

4. Procédé selon l'une des revendications 1 à 3, caractérisé par le fait qu'on introduit le sel pentasodique de l'acide diéthylène triamine pentaacétique sous la forme d'une solution aqueuse.

5. Procédé selon l'une des revendications 1 à 4, caractérisé par le fait que l'on conduit la réaction sous une pression de 2 à 9 bars, en particulier de 3 à 6 bars.

6. Procédé selon l'une des revendications 1 à 5, caractérisé par le fait que l'on conduit la réaction à une température de 35 à 65°C, en particulier de 40 à 55°C.

7. Procédé selon l'une des revendications 1 à 6, caractérisé par le fait que l'on conduit la réaction avec un rapport molaire du CH₃-Cl au méthacrylate de N,N-diméthylaminoéthyle de 1 à 1,1, de préférence de 1,01 à 1,05.

8. Procédé selon l'une des revendications 1 à 7, caractérisé par le fait qu'au moins un stabilisant, choisi notamment parmi le 3,5-ditert.-butyl-4-hydroxytoluène, l'éther méthylique de l'hydroquinone, l'hydroquinone, le catéchol, le tert.-butyl catéchol et leurs mélanges, est associé au méthacrylate de N,N-diméthylaminoéthyle dès le début de la réaction, à raison de 50 à 2000 ppm, de préférence de 100 à 1000 ppm, par rapport à la solution aqueuse de sel (I).

9. Procédé selon l'une des revendications 1 à 8, caractérisé par le fait que :
(a) dans un réacteur fermé, qui contient le méthacrylate de N,N-diméthylaminoéthyle et le sel pentasodique de l'acide diéthylène triamine pentaacétique et qui a été pressurisé par de l'air de 1 à 3 bars, on introduit en continu, à la température de 40 - 55°C, 5 à 15% de la quantité pondérale nécessaire à la réaction de CH₃-Cl ;
(b) ensuite, on ajoute en continu, à la température de 45 - 55°C, l'eau et le reste de CH₃-Cl jusqu'à l'obtention de la concentration souhaitée en sel (I) dans l'eau, la pression en fin de réaction pouvant atteindre 6 - 9 bars ; puis
(c) on dépressurise le réacteur et, après retour à la pression atmosphérique, on élimine le CH₃-Cl résiduaire par exemple par strippage à l'air.

10. Solutions aqueuses obtenues par le procédé tel que défini à l'une des revendications 1 à 9.

## Patentansprüche

1. Verfahren zur Herstellung einer wäßrigen Lösung des ungesättigten quartären Ammoniumsalzes der folgenden Formel (I) 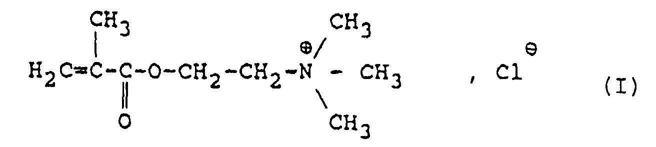 durch Umsetzung unter Druck und in Gegenwart von Wasser von N,N-Dimethylaminoethylmethacrylat mit CH₃-Cl als Quaternisierungsmittel,
dadurch gekennzeichnet, daß
es in Gegenwart des Pentanatriumsalzes der Diethylentriaminpentaessigsäure durchgeführt wird.

2. Verfahren nach Anspruch 1, dadurch gekennzeichnet, daß das Pentanatriumsalz der Diethylentriaminpentaessigsäure in einem Anteil von 5 bis 30 ppm, bezogen auf die wäßrige Lösung des Salzes (I), zugegeben wird.

3. Verfahren nach einem der Ansprüche 1 und 2, dadurch gekennzeichnet, daß das Pentanatriumsalz der Diethylentriaminpentaessigsäure zu Beginn der Umsetzung zugegeben wird.

4. Verfahren nach einem der Ansprüche 1 bis 3, dadurch gekennzeichnet, daß das Pentanatriumsalz der Diethylentriaminpentaessigsäure in Form einer wäßrigen Lösung zugegeben wird.

5. Verfahren nach einem der Ansprüche 1 bis 4, dadurch gekennzeichnet, daß die Umsetzung bei einem Druck von 2 bis 9 bar, insbesondere 3 bis 6 bar, durchgeführt wird.

6. Verfahren nach einem der Ansprüche 1 bis 5, dadurch gekennzeichnet, daß die Umsetzung bei einer Temperatur von 35 bis 65 °C, insbesondere 40 bis 55 °C, durchgeführt wird.

7. Verfahren nach einem der Ansprüche 1 bis 6, dadurch gekennzeichnet, daß die Umsetzung mit einem Molverhältnis CH₃-Cl zu N,N-Dimethylaminoethylmethacrylat von 1 bis 1,1, vorzugsweise 1,01 bis 1,05, durchgeführt wird.

8. Verfahren nach einem der Ansprüche 1 bis 7, dadurch gekennzeichnet, daß mindestens ein Stabilisierungsmittel, das insbesondere unter 3,5-Di-*tert*.-butyl-4-hydroxytoluol, Hydrochinonmethylether, Hydrochinon, Brenzcatechin, *tert*.-Butylbrenzcatechin und Gemischen dieser Verbindungen ausgewählt wird, schon von Beginn der Umsetzung an in einem Mengenanteil von 50 bis 2000 ppm, vorzugsweise 100 bis 1000 ppm, bezogen auf die wäßrige Lösung des Salzes (I), mit dem N,N-Dimethylaminoethylmethacrylat kombiniert wird.

9. Verfahren nach einem der Ansprüche 1 bis 8, dadurch gekennzeichnet, daß
(a) in einen geschlossenen Reaktor, der das N,N-Dimethylaminoethylmethacrylat und das Pentanatriumsalz der Diethylentriaminpentaessigsäure enthält und der durch Aufpressen von Luft unter einem Druck von 1 bis 3 bar steht, kontinuierlich bei einer Temperatur von 40-55 °C 5 bis 15 % der gewichtsbezogenen Menge an CH₃-Cl, die für die Umsetzung von CH₃-Cl erforderlich sind, gegeben werden,
(b) anschließend kontinuierlich bei der Temperatur von 40-55 °C das Wasser und das restliche CH₃-Cl zugegeben werden, bis die gewünschte Konzentration an Salz (I) in dem Wasser erreicht ist, wobei der Druck am Ende der Umsetzung 6 - 9 bar erreichen kann,
(c) dann der Druck im Reaktor auf Atmosphärendruck gesenkt wird und nach Rückkehr des Drucks auf Atmosphärendruck das verbleibende CH₃-Cl beispielsweise durch Strippen mit Luft entfernt wird.

10. Wäßrigen Lösungen, die durch das wie in einem der Ansprüche 1 bis 9 definierte Verfahren erhältlich sind.

## Claims

1. Process for the manufacture of an aqueous solution of the unsaturated quaternary ammonium salt corresponding to the following formula (I): 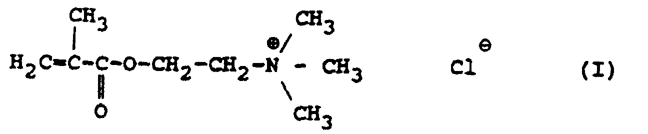 by reaction, under pressure and in the presence of water, of N,N-dimethylaminoethyl methacrylate with CH₃-Cl as quaternizing agent,
characterized in that it is carried out in the presence of the pentasodium salt of diethylenetriaminepentaacetic acid.

2. Process according to Claim 1, characterized in that the pentasodium salt of diethylenetriaminepentaacetic acid is introduced in the proportion of 5 to 30 ppm with respect to the aqueous solution of salt (I).

3. Process according to one of Claims 1 and 2, characterized in that the pentasodium salt of diethylenetriaminepentaacetic acid is introduced at the beginning of the reaction.

4. Process according to one of Claims 1 to 3, characterized in that the pentasodium salt of diethylenetriaminepentaacetic acid is introduced in the form of an aqueous solution.

5. Process according to one of Claims 1 to 4, characterized in that the reaction is carried out under a pressure of 2 to 9 bar, in particular of 3 to 6 bar.

6. Process according to one of Claims 1 to 5, characterized in that the reaction is carried out at a temperature of 35 to 65°C, in particular of 40 to 55°C.

7. Process according to one of Claims 1 to 6, characterized in that the reaction is carried out with a molar ratio of CH₃-Cl to N,N-dimethylaminoethyl methacrylate of 1 to 1.1, preferably of 1.01 to 1.05.

8. Process according to one of Claims 1 to 7, characterized in that at least one stabilizing agent, chosen in particular from 3,5-di-tert-butyl-4-hydroxytoluene, hydroquinone methyl ether, hydroquinone, catechol, tert-butylcatechol and their mixtures, is used in combination with the N,N-dimethylaminoethyl methacrylate from the beginning of the reaction, in the proportion of 50 to 2000 ppm, preferably of 100 to 1000 ppm, with respect to the aqueous solution of salt (I).

9. Process according to one of Claims 1 to 8, characterized in that:
(a) 5 to 15% by weight of the amount of CH₃-Cl necessary for the reaction are continuously introduced, at a temperature of 40-55°C, into a closed reactor which contains the N,N-dimethylaminoethyl methacrylate and the pentasodium salt of diethylenetriaminepentaacetic acid and which has been pressurized with air from 1 to 3 bar;
(b) water and the remainder of the CH₃-Cl are then continuously added, at a temperature of 45-55°C, until the desired concentration of salt (I) in the water is obtained, it being possible for the pressure at the end of the reaction to reach 6-9 bar; then
(c) the reactor is depressurized and, after returning to atmospheric pressure, the residual CH₃-Cl is removed, for example by stripping with air.

10. Aqueous solutions obtained by the process as defined in one of Claims 1 to 9.
